# EUROPÄISCHE PATENTSCHRIFT

(11) **EP 1 796 611 B1**
(45) Veröffentlichungstag und Bekanntmachung des Hinweises auf die Patenterteilung: **13.08.2008**
(21) Anmeldenummer: 05797308.3
(22) Anmeldetag: 04.10.2005
(51) Int. Cl.: A61F 13/64, A61F 13/56

(54) **WEGWERFWINDEL, INSBESONDERE ZUR INKONTINENTENVERSORGUNG**
DISPOSABLE NAPPY IN PARTICULAR FOR INCONTINENT CARE
COUCHE JETABLE, EN PARTICULIER POUR PERSONNES INCONTINENTES

(30) Priorität: 06.10.2004 DE 102004048540
(43) Veröffentlichungstag der Anmeldung: 20.06.2007
(73) Patentinhaber: Paul Hartmann AG, 89522 Heidenheim (DE)
(72) Erfinder: BANDORF, Matthias, 97422 Schweinfurt (DE); BENNING, Markus, 89547 Gerstetten (DE); HALBAUER, Peter, 89547 Gerstetten (DE)
(74) Vertreter: Friz, Oliver
(86) Internationale Anmeldenummer: PCT/EP2005/010648
(87) Internationale Veröffentlichungsnummer: WO 2006/037595

(56) Entgegenhaltungen:
- WO-A-02/22063

## Beschreibung

Die Erfindung betrifft eine Wegwerfwindel, insbesondere zur Inkontinentenversorgung, mit einem Hüftgürtel, wobei der Hüftgürtel einen ersten Hüftgürtelabschnitt mit einem ersten Endabschnitt und einen zweiten Hüftgürtelabschnitt mit einem zweiten Endabschnitt aufweist und wobei der erste und zweite Hüftgürtelabschnitt mittels erster Verschlussmittel zur Bildung einer geschlossenen Hüftöffnung aufeinander festlegbar sind und mit einem einen Vorderbereich, einen Rückenbereich und einen dazwischenliegenden Schrittbereich aufweisenden Windelhauptteil, der einen Absorptionskörper für Flüssigkeiten aufweist, wobei der Windelhauptteil mit dem Längsende seines Vorderbereichs oder seines Rückenbereichs über zweite Verschlussmittel lösbar am Hüftgürtel festlegbar ist, derart, dass ein Benutzer bei angelegtem Hüftgürtel den Windelhauptteil zwischen den Beinen hervorholen und das noch freie Längsende des Windelhauptteils am Hüftgürtel lösbar festlegen kann.

Eine derartige Wegwerfwindel ist beispielsweise aus US 2001/0034512 A1 oder EP 1 269 949 A2 bekannt.

Derartige Wegwerfwindeln bieten den Vorteil, dass ein Benutzer beim Anlegen der Windel zunächst den Hüftgürtel um die Hüften herumlegt, und üblicherweise im Bauchbereich schließen kann. Währenddessen hängt der üblicherweise mit seinem Rückenbereich am Gürtel befestigte Windelhauptteil der Wegwerfwindel lose nach unten. Der Benutzer ergreift dann nach dem Schließen des Hüftgürtels das freihängende Ende des Windelhauptteils und führt den Windelhauptteil von hinten zwischen den Beinen hervor, um den Windelhauptteil mit seinem freien Längsende am Hüftgürtel innen oder außen lösbar festzulegen. Hierfür sind die ersten Verschlussmittel vorgesehen. Es versteht sich, dass das Anlegen der Wegwerfwindel auch so ausgeführt werden kann, dass nach dem Anlegen und Schließen des Hüftgürtels der frei nach unten hängende Windelhauptteil von vom nach hinten zwischen den Beinen eines Benutzers hindurchgeführt und solchenfalls mit seinem Rückenbereich am Hüftgürtel lösbar befestigt werden kann. Es sind aber auch Wegwerfwindeln bekannt geworden, bei denen der Windelhauptteil ganz vom Hüftgürtel lösbar ist, so dass insbesondere bei stark pflegebedürftigen und immobilen Benutzern eine weitgehende Flexibilität bei der Handhabung der Wegwerfwindel gewährleistet ist.

Die gattungsgemäßen Wegwerfwindeln finden Ihre Verwendung vor allem bei Erwachsenen. Erwachsene weisen im Vergleich zu Babys und Kleinkindern ein höheres Maß an Variabilität des Hüftumfanges auf. Damit geht das Problem der mangelnden Flexibilität dieser Wegwerfwindeln hinsichtlich der Anpassung der Windel an die Länge des Hüftumfanges des Verwenders einher.

Die WO-02/22063-A1 offenbart bereits eine Lösung dieses Problem. Es wird vorgeschlagen, eine der Gürtelhälften derart mit Haftmitteln zu versehen, dass diese eingefaltet werden kann und die Bildung der geschlossenen Hüftöffnung ausschließlich durch die andere der beiden Gürtelhälften erfolgt.

Dies Lösung ist insofern nachteilig als zum einen der Vorgang des Einfaltens der einen Gürtelhälfte vom Verwender als sehr mühselig empfunden wird. Außerdem trägt die gefaltete Gürtelhälfte auf. Auch verursachen die Faltkanten Druckstellen, insbesondere bei bettlägerigen Verwendern.

Nicht zuletzt ist die gewünschte Flexibilität der Längenanpassung nur geringfügig verbessert, da die Windel insbesondere nur für sehr große und sehr kleine Hüftumfänge anpassbar ist.

Der vorliegenden Erfindung liegt die Aufgabe zugrunde, eine wegwerfwindel der gattungsgemäßen Art im Hinblick auf das vorgenannte Problem zu verbessern insbesondere die Flexibilität der Längenanpassung zu erhöhen und dem Verwender hierbei Handlingvorteile gegenüber den bekannten Lösungen zu verschaffen.

Diese Aufgabe wird bei einer Wegwerfwindel der genannten Art erfindungsgemäß dadurch gelöst, dass zumindest einer der Hüftgürtelabschnitte ein Trennungsmittel aufweist, mithilfe dessen zumindest ein Endabschnitt abtrennbar ist, um auf einfache weise die Länge des Gürtels verschiedenen Hüftumfängen anpassen zu können.

Damit ist die Länge zumindest einer der beiden Hüftgürtelabschnitte also verringerbar, so dass für den Verwender eine sehr einfache Möglichkeit geschaffen ist, die durch den geschlossenen Hüftgürtel gebildete Hüftöffnung an den eigenen Hüftumfang anzupassen.

In einer vorteilhaften Ausführungsform der Erfindung ist die Erstreckung des ersten Hüftgürtelabschnittes im entfalteten Zustand in Querrichtung über den Längsrand des Hauptteils hinaus größer, vorzugsweise um 100 mm größer, besonders bevorzugt um 200 mm größer, weiter bevorzugt um 300 mm größer und ganz besonders bevorzugt um 400 mm größer als die des zweiten Hüftgürtelabschnittes. Solchenfalls ist es vorteilhaft, dass zumindest und vorzugsweise ausschließlich dieser erste Hüftgürtelabschnitt das oder die Trennmittel aufweist.

Vorteilhafterweise sind die ersten Verschlussmittel durch ein klebendes oder mechanisches Element gebildet, die am Ende insbesondere des zweiten Hüftgürtelabschnittes angeordnet sind. In besonders vorteilhafter Weise sind die ersten Verschlussmittel durch ein Kletthakentape gebildet. Ganz besonders bevorzugt kann dieses Kletthakentape zur Fixierung der geschlossenen Hüftöffnung mit zumindest einem Teil der Außenseite des ersten Hüftgürtelabschnittes in Eingriff gebracht werden.

Das Trennmittel kann an sich beliebig ausgebildet sein, insofern es geeignet ist, einen Endabschnitt eines Hüftgürtelabschnittes abtrennbar zu gestalten. Als vorteilhaft haben sich insbesondere Schwächungslinien, wie Perforationen oder Dünnstellen erwiesen. Aber auch von Verpackungsmitteln bekannte Aufreißfäden erweisen sich als geeignet. Ebenso vorteilhaft kann das den Hüftgürtel bildende Material eine insbesondere linienförmige Schwächung mittels thermischer Behandlung erfahren haben. Der Trennungsmittel kann in einfacher Weise auch durch eine kleine Kerbe gebildet sein, die das Einreißen des Hüftgürtelabschnittes erleichtert.

In Weiterbildung des Erfindungsgedankens wird vorgeschlagen einen oder beide Hüftgürtelabschnitte mit mehreren Trennmitteln der vorgenannten Art zu versehen, um die Flexibilität der Längenanpassung weiter zu erhöhen.

Als vorteilhaft hat sich herausgestellt, wenn der zumindest eine abtrennbare Endabschnitt eine Erstreckung in Windelquerrichtung von mindestens 20mm, vorzugsweise von 30mm-800mm, besonders bevorzugt von 50mm-600mm, ganz besonders bevorzugt von 60mm-500mm, insbesondere vorzugsweise von 70mm-400mm und weiter insbesondere von 90 mm-250 mm besitzt. Es versteht sich, dass, falls mehrere abtrennbare Abschnitte vorgesehen sind, diese von gleicher oder verschiedener Quererstreckung sein können.

Vorteilhafterweise ist der Hüftgürtel der erfindungsgemäßen Windel von einem einstückigen Materialabschnitt gebildet, der an den Windelhauptteil lösbar oder unlösbar angefügt ist. Aufgrund der flächenhaften Anfügung werden die Zugkräfte dann nicht in den Windelhauptteil eingeleitet sondern können ganz von dem reißfesten Gürtelmaterial aufgenommen werden. Dies eröffnet bei der Auswahl der den Hauptteil bildenden Chassismaterialien eine größere Flexibilität. Außerdem sind die Anforderungen an die Stabilität der Anfügung des Gürtels an den Hauptteil geringer. Der den Gürtel bildende Materialabschnitt kann, als ein einziger Längsabschnitt einer in der Maschinenrichtung zugeführten Flachmaterialbahn erhalten werden.

In weiterer Ausbildung des Erfindungsgedankens erweist es sich als vorteilhaft, wenn der Hüftgürtel beidseits um in Längsrichtung der Windel erstreckte Falzlinien auf sich selbst gefaltet ist. Vorteilhafterweise ist der Hüftgürtel in dieser beidseits gefalteten Konfiguration lösbar fixiert, so dass er sich innerhalb der schnelllaufenden Herstellungsmaschine nicht unbeabsichtigt entfaltet oder aufflattert. Die Faltung und lösbare Fixierung des Hüftgürtels in der gefalteten Konfiguration ermöglicht in vorteilhafte Weiser die Anfügung des sehr langen Hüftgürtels an den Hauptteil in der Herstellungsmaschine.

In Weiterbildung dieses Erfindungsgedankens erweist es sich als vorteilhaft, wenn die lösbare Fixierung durch Fügestellen oder Fügebereiche zwischen den aufeinandergefalteten Teilabschnitten des den Hüftgürtel bildenden Materialabschnitts gebildet ist. Es wäre aber auch möglich, dass die gefaltete Konfiguration durch sonstige Haltemittel, etwa einen lösbaren Tapeabschnitt, lösbar fixiert ist.

Die vorerwähnte und vorzugsweise in einem Zug zu lösende Fixierung der aufeinander gefalteten Teilabschnitte des an den Hauptteil angefügten den Hüftgürtel bildenden Materialabschnitts kann beispielsweise durch kalte Verprägung, oder durch Verprägung unter Anwendung von Temperatur (Thermoverschweißung), durch Vernadelung, insbesondere Heißvernadelung, oder durch Ultraschallverschweißung oder Laserverschweißung oder ähnliche gleichwirkende Fügemethoden erreicht werden.

Im einfachsten Fall ist der Materialabschnitt beidseits der Längsmittelachse um eine Falzlinie auf sich selbst gefaltet, so dass zwei Teilabschnitte aufeinander liegen bzw. gegeneinander anliegen. Vorzugsweise ist der Materialabschnitt aber um wenigstens zwei Falzlinien auf sich selbst gefaltet, so dass eine im Schnitt Z-förmige Konfiguration entsteht. Nach einer weiteren bevorzugten Ausführungsform sind die Materialabschnitte um drei Falzlinien auf sich selbst gefaltet. Nach einer weiteren bevorzugten Ausführungsform sind die Materialabschnitte um vier Falzlinien auf sich selbst gefaltet.

Nach einer weiteren bevorzugten Ausführungsform der erfindungsgemäßen Gürtelwindel steht der Hüftgürtel, insbesondere der einstückige Materialabschnitt des Hüftgürtels, in gefalteter Konfiguration mit einem Anfassbereich über einen Längsseitenrand des Windelhauptteils in Querrichtung vor. Der Anfassbereich kann insbesondere vom jeweiligen freien Ende des Hüftgürtels gebildet sein.

Vor der Entfaltung des einstückigen Materialabschnitts sind die Anfassbereiche vorzugsweise in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind. Die Gürtelwindel erweist sich insbesondere bei der Anwendung bei stark pflegebedürftigen Personen als besonders vorteilhaft. So wird die Gürtelwindel beispielsweise häufig an pflegebedürftige Patienten angelegt, während diese sich in Seitenlage befinden. Hierbei muss der über den Hauptteil seitlich überstehende Materialabschnitt unter dem Patienten hindurch geführt werden. Dieser Vorgang des unter dem Patienten Hindurchführens ist mit dem lösbar fixierten gefalteten Materialabschnitt, welcher den Gürtel bildet, nun wesentlich vereinfacht.
Die lösbare Fixierung der aufeinander gefalteten Teilabschnitte des Materialabschnitts aneinander und möglicherweise auch an dem Hauptteil ist vorzugsweise durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Hauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt- oder Kreisform abweichende Formen wie Dreieck-, Viereck-, Vieleck-, oder Ovalformen. Vorzugsweise ist die lösbare Fixierung der aufeinander gefalteten Teilabschnitte der Materialabschnitte aneinander durch thermisch oder durch Ultraschall erzeugte, vorzugsweise punktförmige Fügestellen ausgebildet.

Die Erstreckung des den Hüftgürtel bildenden Materialabschnitts im entfalteten Zustand in Querrichtung über den Längsrand des Hauptteils hinaus beträgt wenigstens 200 mm, insbesondere wenigstens 300 mm, insbesondere wenigstens 400 mm, insbesondere wenigstens 500 mm und weiter insbesondere wenigstens 600 mm und weiter insbesondere wenigstens 700 mm und besonders bevorzugt wenigstens 800 mm.

Seine Erstreckung in Längsrichtung beträgt 30 bis 120 mm, insbesondere 30 bis 100 mm, insbesondere 30 bis 80 mm, insbesondere 30 bis 75 mm, insbesondere von 44 bis 70 mm, insbesondere von 40 bis 65 mm und weiter insbesondere von 40 bis 60 mm aufweist.

Der einstückige Materialabschnitt ist in vorteilhafter Weise unlösbar an eine Außenseite des Hauptteils angefügt. Diese Anfügung kann in an sich beliebiger Weise ausgeführt sein. Der einstückige Materialabschnitt kann indessen in vorteilhafter Weise mittels eines nur bereichsweisen Kleberauftrags an den Windelhauptteil angefügt sein. Insbesondere erweist es sich als vorteilhaft, wenn der Kleber nicht bis zu dem Rand der gegeneinander anliegenden flächenhaften Erstreckung des Materialabschnitts und des Hauptteils aufgebracht ist, so dass ein Randumfangsbereich dieses Verbunds kleberfrei bleibt. Dies hat den Vorteil, dass solchenfalls beim Laminieren kein Kleber zwischen den Lagen nach außen gedrückt wird.

Die vorliegende Erfindung hat auch ein Verfahren zum Herstellen einer Wegwerfwindel der erfindungsgemäßen Art zum Gegenstand. Dieses Verfahren ist gekennzeichnet durch die folgenden Verfahrensschritte:
- Zuführen einer endlosen Flachmaterialbahn zur Bildung von Materialabschnitten für die Herstellung des Hüftgürtels,
- Falten der Flachmaterialbahn um in Längsrichtung verlaufende Falzlinien, wobei die Flachmaterialbahn beidseits einer Längsmittelachse auf sich selbst gefaltet wird,
- Abtrennen von Längsabschnitten der gefalteten Flachmaterialbahn zur jeweiligen Bildung eines einstückigen Materialabschnitts für die Herstellung des Hüftgürtels und
- unlösbares Fixieren des Materialabschnitts an einem Windelhauptteil.

Bevorzugte Weiterbildungen des erfindungsgemäßen Verfahrens ergeben sich aus den weiteren Unteransprüchen. Insbesondere erweist es sich als vorteilhaft, wenn der einstückige den Hüftgürtel bildende Materialabschnitt mittels Kleber an den Windelhauptteil angefügt wird. Es erweist sich des Weiteren als vorteilhaft, wenn hierfür der Kleber auf die Flachmaterialbahn, von der die Materialabschnitte abgetrennt werden, in voneinander in Längsrichtung beabstandeten Bereichen aufgebracht wird. Ein jeweiliger Schnittbereich wird dabei vorzugsweise kleberfrei gehalten.

Des Weiteren erweist es sich als vorteilhaft, dass der Kleber derart bereichsweise auf die Flachmaterialbahn aufgebracht wird, dass er in Längsrichtung und vorzugsweise auch in Querrichtung von einem Rand der später abgetrennten Flachmaterialabschnitte beabstandet ist. Solchenfalls erweist es sich als vorteilhaft, wenn der Kleber getaktet auf die Flachmaterialbahn aufgebracht wird.

Der einstückige an den Hauptteil angefügte Materialabschnitt ist vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können verwendet werden. Die Verbindung der einzelnen Lagen kann durch an sich übliche und bekannte Verfahren, beispielsweise durch thermische Fügeverfahren (Verschweißung, insbesondere Laserverschweißung, hotmelt, air-through) oder durch Ultraschallschweißverfahren erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien ist denkbar. Auch Filme aus thermoplastischen und insbesondere elastischen Materialien sind verwendbar, und zwar auch als mehrlagige Filmlaminate. Vorteilhafterweise können auch Vlies/Folienlaminate zum Einsatz kommen, die wenigstens eine Filmlage und wenigstens eine Vlieslage oder wenigstens eine Lage eines textilen Materials umfassen. Filmlagen werden insbesondere zur Realisierung von elastischen Bereichen des Hüftgürtels eingesetzt. Die Verbindung der Lagen kann wiederum durch die vorgenannten Verfahren erfolgen.

Vorzugsweise wird der Materialabschnitt zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Die Materialabschnitte haben in vorteilhafter Weise ein Flächengewicht von 20 bis 150 g/m², insbesondere 30-100 g/m² und bevorzugt 40-80 g/m².

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der erfindungsgemäßen Windel. In der Zeichnung zeigt:
- Figur 1: eine schematische Darstellung einer erfindungsgemäßen Gürtelwindel mit entfaltetem Hüftgürtel;
- Figur 2: eine schematische Darstellung einer erfindungsgemäßen Gürtelwindel mit gefaltetem Hüftgürtel;
- Figur 3: eine schematische Schnittansicht mit Schnittebene A-A der Gürtelwindel nach Figur 2 mit einer ersten Faltung des Hüftgürtels;
- 4a - e: in schematischer Darstellung Zuführung, Faltung, Fixierung, Kleberbeschichtung und Schneiden einer Hüftgürtelabschnitte bildenden Flachmaterialbahn.

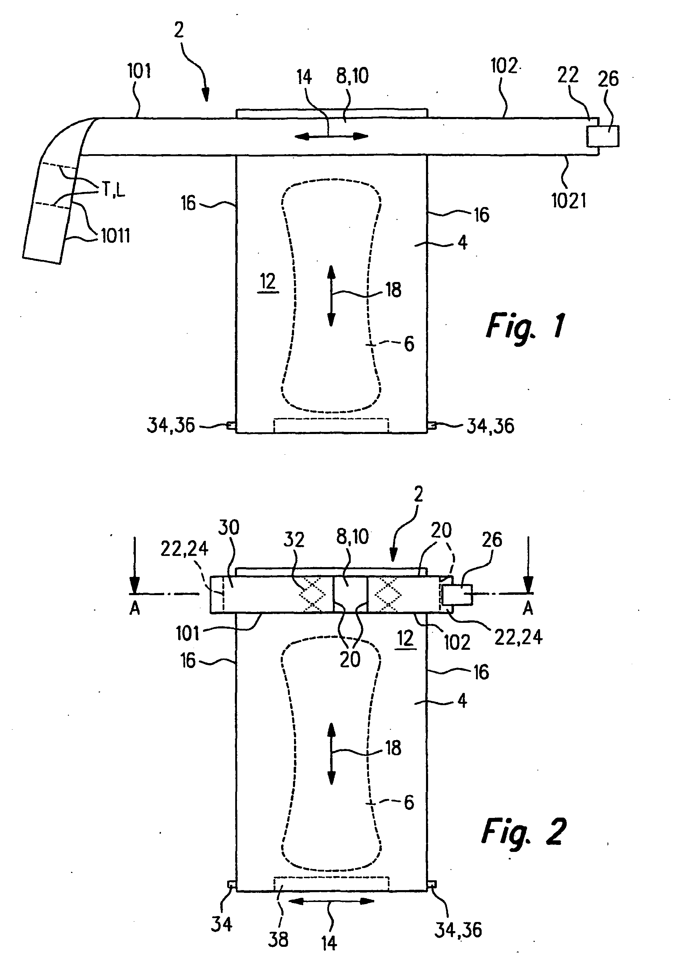

Figuren 1 und 2 zeigen schematisch eine wegwerfbare Gürtelwindel 2 mit einem Hauptteil 4 und einem angedeuteten Absorptionskörper 6. An den Hauptteil 4 angefügt ist ein einstückiger Materialabschnitt 8, welcher einen Hüftgürtel 10 der Gürtelwindel bildet. Figur 1 zeigt den Gürtel in entfaltetem Zustand während Figur 2 den Gürtel in gefaltetem Zustand veranschaulicht. In Figur 1 sind an einem ersten über den Seitenrand des Hauptteils 4 überstehenden Hüftgürtelabschnitt 101 zwei voneinander beabstandete Trennmittel T in Form von Perforationslinien L erkennbar. Der einstückige Materialabschnitt 8 ist an eine Außenseite 12 des Hauptteils 4 auf noch näher zu beschreibende Weise unlösbar angefügt. Er erstreckt sich in Querrichtung 14 der Gürtelwindel 2 über seitliche Längsränder 16 im entfalteten Zustand jeweils über wenigstens 300 mm; insbesondere über wenigstens 400 mm, insbesondere über wenigstens 500 mm, insbesondere über wenigstens 600 mm hinaus. Im dargestellten Fall der Figur 1 ist der erste Materialabschnitt 101 deutlich länger als der zweite Materialabschnitt 102, das heißt die Erstreckung des ersten Hüftgürtelabschnittes 101 im entfalteten zustand in Querrichtung 14 über den Längsrand 16 des Hauptteils 4 hinaus ist größer, vorzugsweise um 100mm größer, besonders bevorzugt um 200 mm größer ganz besonders bevorzugt um 300mm größer als die des zweiten Hüftgürtelabschnittes 102.

In Figur 2 ist der einstückige Materialabschnitt 8 beidseits einer Windellängsrichtung 18 um Falzlinien 20 mehrfach auf sich selbst gefaltet. Das jeweils freie Ende 22 des Materialabschnitts 8 bildet einen über die Längsseitenränder 16 um wenigstens 10 mm vorstehenden Anfassbereich 24 zum Ergreifen mit den Fingern eines Benutzers. Am ersten Hüftgürtelabschnitt 102 ist an dessen Ende 22 auch ein Verschlusselement 26 vorgesehen, und zwar beispielsweise in Form einer klebenden oder vorzugsweise mechanischen Verschlusselemente aufweisenden Lasche, welche mit einem Auftreffbereich oder mit Gegenverschlusselementen am ersten Hüftgürtelabschnitt 101, insbesondere mit dessen gesamter Außenfläche lösbar haftend zusammenwirken kann, wenn der Hüftgürtel 10 zur Bildung einer in Hüftumfangsrichtung geschlossenen Hüftöffnung geschlossen wird. Vorteilhafterweise umfasst die Außenseite des Hüftgürtels ein Fasermaterial wie beispielsweise ein Textil oder einen Vliesstoff, der direkt mit dem Verschlusselement 26 in Eingriff bringbar ist.

Die aufeinander gefalteten Teilabschnitte 30 des einstückigen Materialabschnitts 8 sind lösbar aufeinander fixiert, und zwar durch eine Anzahl von im Wesentlichen punktförmigen Fügestellen 32 in Form von vorzugsweise Ultraschallschweißpunkten. Hierfür sind einige wenige Fügestellen ausreichend.

Zum Anlegen der Gürtelwindel 2 wird der Hüftgürtel 10 entfaltet. Zur Längenanpassung kann anschließend ein Endabschnitt 1011 des ersten Hüftgürtelabschnittes 101 entweder entlang der ersten oder der zweiten Perforationslinie L abgetrennt werden.

Anschließend wird der Hüftgürtel über das Verschlusselement 26 des zweiten Hüftgürtelabschnittes 102 und den entsprechend ausgebildeten Auftreffbereich am ersten Hüftgürtelabschnitt 101 auf sich selbst geschlossen. Der Benutzer oder eine Pflegeperson holt nun das im allgemeinen frei nach unten hängende Ende des Hauptteils zwischen den Beinen des Benutzers hervor und befestigt es lösbar an dem ringförmig geschlossenen Hüftgürtel 10, und zwar vorzugsweise auf der vom Benutzer abgewandten Außenseite des Hüftgürtels 10. Der Hauptteil 4 kann wiederum über an sich beliebige klebend oder mechanisch wirkende Verschlussmittel 34, im dargestellten Fall über seitlich vom Hauptteil vorstehende Verschlusslaschen 36 lösbar festgelegt werden. Zusätzlich können in einem entsprechenden Überdeckungsbereich 38, vorzugsweise an der körperzugewandten Innenseite des Hauptteils 4, Verschlussmittel im weitesten Sinn und in an sich beliebiger Ausführung vorgesehen sein.

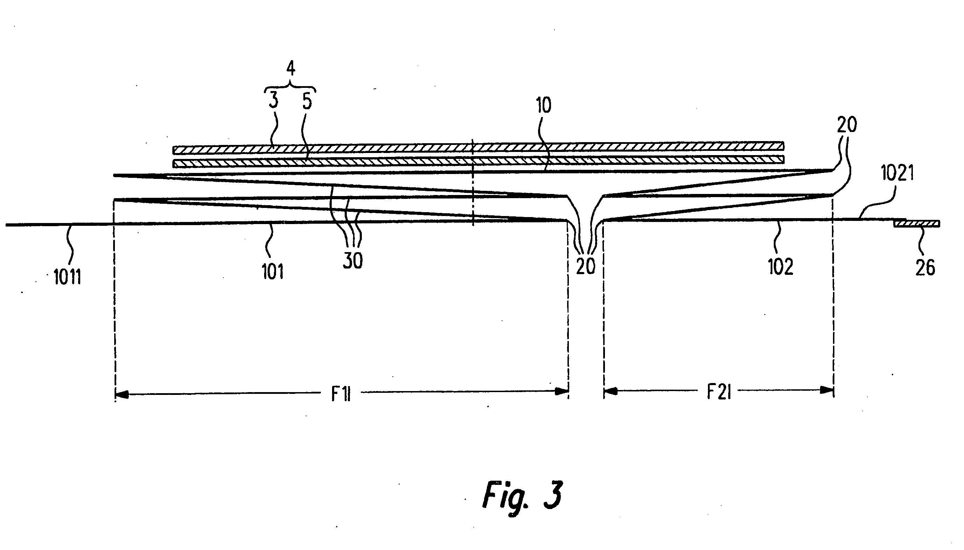

Die Figur 3 zeigt eine besonders bevorzugte Faltung des einstückigen Materialabschnitts 8, der außen, also körperabgewandt, am Ende des Windelhauptteils 4 angebracht ist. Der Windelhauptteil wird an seinem Ende im dargestellten Fall durch ein körperseitiges Topsheet 3 und ein wäscheseitiges Backsheet 5 gebildet. Die Faltung umfasst auf jeder Seite vier Falzlinien 20; eine jeweilige Faltung ist also doppel-Z-förmig. Der Gürtel ist asymmetrisch gefaltet. Die Faltbreite (F1,1) des ersten längeren Hüftgürtelabschnitts 101 ist größer als die Faltbreite (F2,1) des kürzeren zweiten Hüftgürtelabschnitts 102.

Die Gesamtlänge des Hüftgürtels 10 (einschließlich der Quererstreckung über den Hauptteil, also die Umfangslänge der gesamten Hüftöffnung) beträgt ca. 1400 mm (ein etwaiger Überlappungsbereich beim Schließen des Hüftgürtels 10 ist dabei nicht berücksichtigt). Die Gürtelbreite, also deren Erstreckung in Längsrichtung 18, beträgt zwischen 50 und 100 mm.

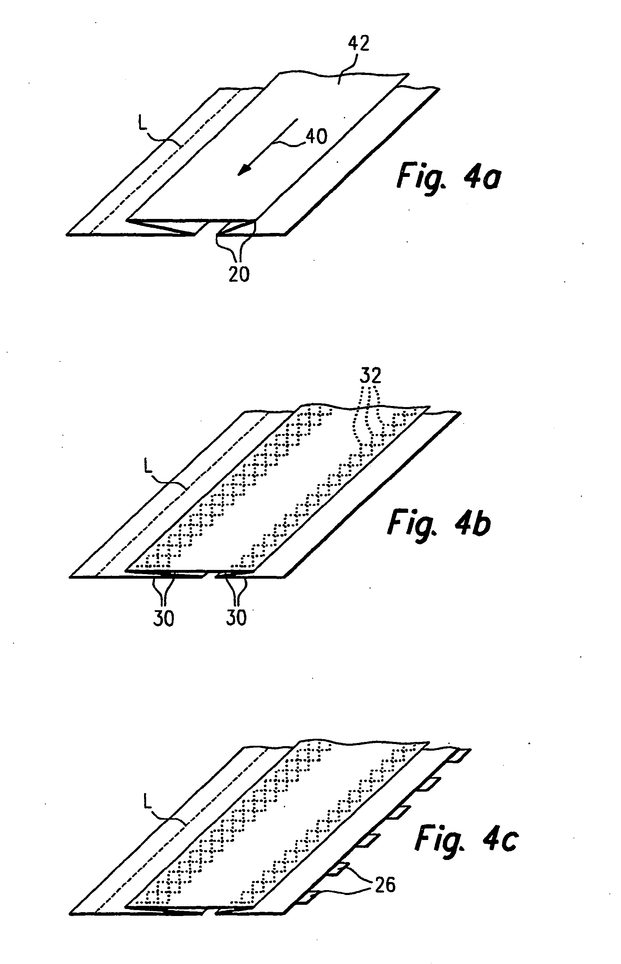

Die Figuren 4a bis 4e verdeutlichen das Herstellungsverfahren für eine erfindungsgemäße Gürtelwindel, und zwar die Herstellung des Hüftgürtels 10. Hierfür wird eine in einer Maschinenlängsrichtung 40 endlose Flachmaterialbahn 42 zugeführt. Die Flachmaterialbahn weist bereits eine Perforationslinie L auf. Es versteht sich, dass das Gürtelmaterial aber auch zu einem späteren Zeitpunkt mit dem Trennmittel versehen werden kann. Die Flachmaterialbahn 42 wird im dargestellten Fall der Figur 4a beispielhaft um zwei Falzlinien 20, also Z-förmig, gefaltet. Auch eine Faltung um drei oder vier oder mehr Falzlinien wäre denkbar. Die aufeinander gefalteten Teilabschnitte 30 werden, wie aus Figur 4b schematisch ersichtlich, lösbar aneinander fixiert. Dies kann in vorteilhafter Weise durch im Wesentlichen punktförmige Fügestellen 32, insbesondere durch Ultraschallschweißpunkte, ausgeführt werden. Figur 4c zeigt die Anfügung von Verschlusselementen 26 an einer Seite der gefalteten Flachmaterialbahn 42.

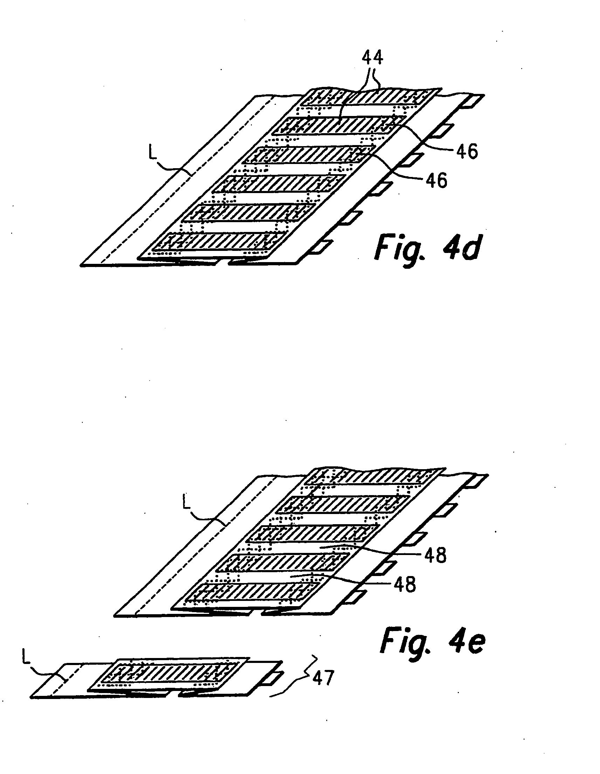

Sodann wird, wie aus Figur 4d ersichtlich ist, ein bereichsweiser Kleberauftrag 44 in in Längsrichtung 40 voneinander beabstandeten Bereichen 46 der Flachmaterialbahn 42 in einem vorzugsweise getakteten Verfahren aufgebracht.

Wie Figur 4e verdeutlicht, werden dann Längsabschnitte 47 quer zur Längsrichtung 40 von der endlos zugeführten Flachmaterialbahn 42 abgetrennt, welche den in Querrichtung einstückig durchgehenden Materialabschnitt 8 zur Bildung des Hüftgürtels 10 darstellen. Man erkennt aus Figur 4e, dass der Schnitt durch kleberfreie Bereiche 48 geführt wird.

Der so abgetrennte Längsabschnitt 47 der Flachmaterialbahn 42 wird dann vorzugsweise auf die Außenseite 12 eines Windelhauptteils 4 unlösbar aufgebracht, so wie dies in Figur 1 dargestellt ist und bereits beschrieben wurde.

## Patentansprüche

1. Wegwerfwindel (2), insbesondere zur Inkontinentenversorgung, mit einem Hüftgürtel (10), wobei der Hüftgürtel (10) einen ersten Hüftgürtelabschnitt (101) mit einem ersten Endabschnitt (1011) und einen zweiten Hüftgürtelabschnitt (102) mit einem zweiten Endabschnitt (1021) aufweist und wobei der erste und zweite Hüftgürtelabschnitt mittels erster Verschlussmittel (26) zur Bildung einer geschlossenen Hüftöffnung aufeinander festlegbar sind und mit einem einen Vorderbereich, einen Rückenbereich und einen dazwischenliegenden Schrittbereich aufweisenden Windelhauptteil (4), der einen Absorptionskörper (6) für Flüssigkeiten aufweist, wobei der Windelhauptteil (4) mit dem Längsende seines Vorderbereichs oder seines Rückenbereichs über zweite Verschlussmittel (34) lösbar am Hüftgürtel (10) festlegbar ist, derart, dass ein Benutzer bei angelegtem Hüftgürtel (10) den Windelhauptteil (4) zwischen den Beinen hervorholen und das noch freie Längsende des Windelhauptteils (4) am Hüftgürtel (10) lösbar festlegen kann, **dadurch gekennzeichnet, dass** zumindest einer der Hüftgürtelabschnitte (101, 102) ein Trennungsmittel (T, L) aufweist, mithilfe dessen zumindest ein Endabschnitt (1011, 1021) abtrennbar ist (um die Länge des Gürtels verschiedenen Hüftumfängen anpassen zu können).

2. Wegwerfwindel nach Anspruch 1, **dadurch gekennzeichnet, dass** die Erstreckung des ersten Hüftgürtelabschnittes (101) im entfalteten Zustand in Querrichtung (14) über den Längsrand (16) des Hauptteils (4) hinaus größer ist, insbesondere um 100 mm größer, insbesondere um 200 mm größer, insbesondere um 300 mm größer und weiter insbesondere um 400 mm größer ist als die des zweiten Hüftgürtelabschnitts (102).

3. Wegwerfwindel nach einem oder mehreren der vorstehenden Ansprüche, **dadurch gekennzeichnet, dass** die Erstreckung mindestens einer der Hüftgürtelabschnitte (101, 102) im entfalteten Zustand in Querrichtung (14) über den Längsrand (16) des Hauptteils (4) hinaus wenigstens 200 mm, insbesondere wenigstens 300 mm, insbesondere wenigstens 400 mm, insbesondere wenigstens 500 mm, insbesondere wenigstens 600 mm, insbesondere wenigstens 700 mm und weiter insbesondere wenigstens 800 mm beträgt.

4. Wegwerfwindel nach einem der vorherigen Ansprüche, **dadurch gekennzeichnet, dass** zumindest einer der Hüftgürtelabschnitte (101, 102) mindestens zwei, vorzugsweise mindestens drei, ganz besonders bevorzugt mindestens vier Trennungsmittel (T, L) aufweisen, so dass mehrere oder unterschiedlich lange Endabschnitte (1011, 1021) abtrennbar sind.

5. Wegwerfwindel nach einem der vorherigen Ansprüche, **dadurch gekennzeichnet, dass** das oder die Trennungsmittel (T, L) durch eine Schwächungslinie (L), insbesondere eine Perforationslinie (L) gebildet ist, deren Richtung eine Komponente in Windellängsrichtung aufweist.

6. Wegwerfwindel nach einem der vorherigen Ansprüche, **dadurch gekennzeichnet, dass** der zumindest eine abtrennbare Endabschnitt (1011, 1021) eine Erstreckung in Windelquerrichtung von mindestens 20mm, vorzugsweise von 30mm-800mm, besonders bevorzugt von 50mm-600mm, ganz besonders bevorzugt von 60mm-500mm, insbesondere vorzugsweise von 70mm-400mm, insbesondere von 80mm-300mm und insbesondere von 90 mm-200 mm aufweist.

7. Wegwerfwindel nach einem der Ansprüche 4-6, **dadurch gekennzeichnet, dass** die abtrennbaren Abschnitte unterschiedlich große Erstreckung in Querrichtung der Windel aufweisen.

8. Wegwerfwindel nach einem der vorherigen Ansprüche, **dadurch gekennzeichnet dass** der Hüftgürtel (10) beidseits um in Längsrichtung (18) der Windel erstreckte Falzlinien (20) auf sich selbst gefaltet ist.

9. Wegwerfwindel nach Anspruch 8, **dadurch gekennzeichnet, dass** die Faltbreite (F1,1) des ersten Hüftgürtelabschnittes (101) größer ist als die Faltbreite (F2,1) des zweiten Hüftgürtelabschnittes (102).

10. Wegwerfwindel nach Anspruch 8 oder 9, **dadurch gekennzeichnet dass** der Hüftgürtel (10) in der gefalteten Konfiguration lösbar fixiert ist.

11. Wegwerfwindel nach Anspruch 10, **dadurch gekennzeichnet, dass** die lösbare Fixierung durch Fügestellen (32) oder Fügebereiche zwischen den aufeinander gefalteten Teilabschnitten (30) des Hüftgürtels gebildet ist.

12. Wegwerfwindel nach Anspruch 11, **dadurch gekennzeichnet, dass** die Fügestellen (32) oder Fügebereiche im Wesentlichen punktförmig sind.

13. Wegwerfwindel nach einem oder mehreren der vorstehenden Ansprüche, **dadurch gekennzeichnet, dass** der Hüftgürtel (10) beidseits um jeweils drei oder vier in Längsrichtung (18) der Windel verlaufende Falzlinien (20) auf sich selbst gefaltet ist.

14. wegwerfwindel nach einem oder mehreren der vorstehenden Ansprüche, **dadurch gekennzeichnet, dass** der Hüftgürtel (10) in gefalteter Konfiguration mit einem Anfassbereich (24) über einen Längsseitenrand (16) des Windelhauptteils (4) in Querrichtung (14) vorstehen.

15. Wegwerfwindel nach einem oder mehreren der vorstehenden Ansprüche, **dadurch gekennzeichnet, dass** der Hüftgürtel (10) eine Erstreckung in Längsrichtung (18) von 30 bis 120 mm, insbesondere von 30 bis 100 mm, insbesondere von 30 bis 80 mm, insbesondere von 30 bis 75 mm, insbesondere von 44 bis 70 mm, insbesondere von 40 bis 65 mm und weiter insbesondere von 40 bis 60 mm aufweist.

16. Wegwerfwindel nach einem oder mehreren der vorstehenden Ansprüche, **dadurch gekennzeichnet, dass** der Hüftgürtel (10) von einem einstückigen Materialabschnitt (8) gebildet ist.

17. Wegwerfwindel nach einem der vorherigen Ansprüche, **dadurch gekennzeichnet dass** der Hüftgürtel unlösbar an eine Außenseite (12) des Hauptteils (4) angefügt ist.

18. Wegwerfwindel nach einem oder mehreren der vorstehenden Ansprüche 16 oder 17, **dadurch gekennzeichnet, dass** der einstückige Materialabschnitt (8) mittels eines insbesondere nur bereichsweisen Kleberauftrags (44) an den Windelhauptteil (4) angefügt ist.

## Claims

1. Disposable diaper (2), in particular, for incontinence care, comprising a hip belt (10), wherein the hip belt (10) has a first hip belt section (101) with a first end section (1011), and a second hip belt section (102) with a second end section (1021), and wherein the first and second hip belt sections can be fixed on top of each other using a first closing means (26) to form a closed hip opening, and with a diaper main part (4) which comprises a front area, a rear area, an intermediate crotch area, and which comprises a liquid absorbing element (6), wherein the longitudinal end of the front area or rear area of the diaper main part (4) can be detachably fixed to the hip belt (10) via second closing means (34), such that the user can grasp the diaper main part (4) from between his/her legs when the hip belt (10) is applied, and can detachably fix the free longitudinal end of the diaper main part (4) to the hip belt (10), **characterized in that** at least one of the hip belt sections (101, 102) has a separating means (T,L) for separating at least one end section (1011, 1021) (in order to adjust the length of the belt to different hip measurements).

2. Disposable diaper according to claim 1, **characterized in that** the transverse extension (14) of the first hip belt section (101) in the unfolded state past the longitudinal edge (16) of the main part (4) is larger than that of the second hip belt section (102), in particular by 100mm, in particular by 200mm, in particular by 300mm, and more particular by 400mm.

3. Disposable diaper according to any one or more of the preceding claims, **characterized in that**, in the unfolded state, at least one of the hip belt sections (101, 102) extends in the transverse direction (14) past the longitudinal edge (16) of the main part (4) by at least 200mm, in particular at least 300mm, in particular at least 400mm, in particular at least 500mm, in particular at least 600mm, in particular at least 700mm and moreover in particular at least 800mm.

4. Disposable diaper according to any one of the preceding claims, **characterized in that** at least one of the hip belt sections (101, 102) comprises at least two, preferably at least three, with particular preference at least four separating means (T, L) such that several end sections or end sections of different lengths (1011, 1021) can be separated.

5. Disposable diaper according to any one of the preceding claims, **characterized in that** the separating means (T, L) is/are formed by a weakening line (L), in particular a perforation line (L), whose direction has a component in the longitudinal direction of the diaper.

6. Disposable diaper according to any one of the preceding claims, **characterized in that** the at least one end section (1011, 1021) that can be separated extends in the transverse direction of the diaper by at least 20mm, preferably 30mm-800mm, in particular 50mm-600mm, with particular preference 60mm-500mm, with particular preference 70mm-400mm, with particular preference 80mm-300mm and preferentially 90mm-200mm.

7. Disposable diaper according to any one of the claims 4 through 6, **characterized in that** the sections that can be separated have different extensions in the transverse direction of the diaper.

8. Disposable diaper according to any one or more of the preceding claims, **characterized in that** the hip belt (10) is folded onto itself on both sides about folding lines (20) that extend in the longitudinal direction (18) of the diaper.

9. Disposable diaper according to claim 8, **characterized in that** the folding width (F1, 1) of the first hip belt section (101) is larger than the folding width (F2, 1) of the second hip belt section (102).

10. Disposable diaper according to claim 8 or 9, **characterized in that** the hip belt (10) is detachably fixed in the folded configuration.

11. Disposable diaper according to claim 10, **characterized in that** the detachable fixing is formed by joints (32) or joining areas between the partial sections (30) of the hip belt, which are folded on top of each other.

12. Disposable diaper according to claim 11, **characterized in that** the joints (32) or joining areas are substantially point-shaped.

13. Disposable diaper according to any one or more of the preceding claims, **characterized in that** the hip belt (10) is folded onto itself on both sides about three or four folding lines (20) in each case that extend in the longitudinal direction (18) of the diaper.

14. Disposable diaper according to any one or more of the preceding claims, **characterized in that**, in the folded configuration, a grasping area (24) of the hip belt (10) projects in a transverse direction (14) past a longitudinal side edge (16) of the diaper main part (4).

15. Disposable diaper according to any one or more of the preceding claims, **characterized in that** the hip belt (10) extends in the longitudinal direction (18) by 30 to 120mm, in particular 30 to 100mm, in particular 30 to 80mm, in particular 30 to 75mm, in particular 44 to 70mm, in particular 40 to 65mm and more particular 40 to 60mm.

16. Disposable diaper according to any one or more of the preceding claims, **characterized in that** the hip belt (10) is formed by a one-piece material section (8).

17. Disposable diaper according to any one or more of the preceding claims, **characterized in that** the hip belt is undetachably joined to an outer side (12) of the main part (4).

18. Disposable diaper according to any one or more of the preceding claims 16 or 17, **characterized in that** the one-piece material section (8) is joined to the diaper main part (4) using an adhesive (44) which is disposed thereon, in particular, only in certain areas.

## Revendications

1. Couche jetable (2), en particulier pour personnes incontinentes, comportant une ceinture de taille (10), la ceinture de taille (10) présentant une première section de ceinture de taille (101) ayant une première section d'extrémité (1011) et une seconde section de ceinture de taille (102) ayant une seconde section d'extrémité (1021) et la première et la seconde sections de ceinture de taille pouvant être fixées l'une sur l'autre au moyen de premiers moyens de fermeture (26) afin de former une ouverture de taille fermée, et comportant une partie principale de couche (4) présentant une zone avant, une zone arrière et une zone d'entrejambe située entre les deux premières, laquelle partie principale de couche possède un corps d'absorption (6) pour liquides, la partie principale de couche (4) pouvant être fixée de manière amovible à la ceinture de taille (10) avec l'extrémité longitudinale de sa zone avant ou de sa zone arrière par l'intermédiaire de seconds moyens de fermeture (34), de telle façon qu'un utilisateur, lorsque la ceinture de taille (10) est posée, puisse amener la partie principale de couche (4) vers l'avant entre les jambes et fixer de manière amovible l'extrémité longitudinale encore libre de la partie principale de couche (4) sur la ceinture de taille (10), **caractérisée en ce qu'**au moins l'une des sections de ceinture de taille (101, 102) présente un moyen de séparation (T, L), à l'aide duquel au moins une section d'extrémité (1011, 1021) peut être séparée (afin de pouvoir adapter la longueur de la ceinture à différents tours de taille).

2. Couche jetable selon la revendication 1, **caractérisée en ce que** l'étendue de la première section de ceinture de taille (101) dans l'état déplié dans le sens transversal (14) au-delà du bord longitudinal (16) de la partie principale (4) est supérieure à la seconde section de ceinture de taille (102), notamment de 100 mm, en particulier de 200 mm, en particulier de 300 mm et en particulier encore de 400 mm.

3. Couche jetable selon l'une ou plusieurs des revendications précédentes, **caractérisée en ce que** l'étendue d'au moins l'une des sections de ceinture de taille (101, 102) dans l'état déplié dans le sens transversal (14) au-delà du bord longitudinal (16) de la partie principale (4) mesure au moins 200 mm, en particulier au moins 300 mm, en particulier au moins 400 mm, en particulier au moins 500 mm, en particulier au moins 600 mm, en particulier au moins 700 mm et en particulier encore au moins 800 mm.

4. Couche jetable selon l'une des revendications précédentes, **caractérisée en ce qu'**au moins l'une des sections de ceinture de taille (101, 102) présente au moins deux, de préférence au moins trois, de façon tout particulièrement préférée au moins quatre moyens de séparation (T, L), de sorte que plusieurs sections d'extrémité ou des sections d'extrémité de longueur différente (1011, 1021) peuvent être séparées.

5. Couche jetable selon l'une des revendications précédentes, **caractérisée en ce que** le ou les moyens de séparation (T, L) sont formés par une ligne de faiblesse (L), en particulier une ligne de perforations (L), dont l'orientation présente une composante dans le sens longitudinal de la couche.

6. Couche jetable selon l'une des revendications précédentes, **caractérisée en ce qu'**au moins une section d'extrémité (1011, 1021) séparable présente une étendue dans le sens transversal de la couche d'au moins 20 mm, de préférence comprise entre 30 et 800 mm, de façon particulièrement préférée comprise entre 50 et 600 mm, de façon tout particulièrement préférée comprise entre 60 mm et 500 mm, en particulier et de préférence comprise entre 70 et 400 mm, en particulier comprise entre 80 et 300 mm et en particulier comprise entre 90 et 200 mm.

7. Couche jetable selon l'une des revendications 4 à 6, **caractérisée en ce que** les sections séparables présentent une étendue de grandeur différente dans le sens transversal de la couche.

8. Couche jetable selon l'une des revendications précédentes, **caractérisée en ce que** la ceinture de taille (10) est repliée sur elle-même des deux côtés autour de lignes de pliage (20) s'étendant dans le sens longitudinal (18) de la couche.

9. Couche jetable selon la revendication 8, **caractérisée en ce que** la largeur de pliage (F1, 1) de la première section de ceinture de taille (101) est supérieure à la largeur de pliage (F2, 1) de la seconde section de ceinture de taille (102).

10. Couche jetable selon la revendication 8 ou 9, **caractérisée en ce que** la ceinture de taille (10) est fixée de manière amovible dans la configuration pliée.

11. Couche jetable selon la revendication 10, **caractérisée en ce que** la fixation amovible est formée par des points d'assemblage (32) ou zones d'assemblage entre les sections partielles (30) de la ceinture de taille pliées les unes sur les autres.

12. Couche jetable selon la revendication 11, **caractérisée en ce que** les points d'assemblage (32) ou zones d'assemblage sont essentiellement sous forme de points.

13. Couche jetable selon l'une ou plusieurs des revendications précédentes, **caractérisée en ce que** la ceinture de taille (10) est repliée sur elle-même des deux côtés autour de trois ou quatre lignes de pliage (20) s'étendant dans le sens longitudinal (18) de la couche.

14. Couche jetable selon l'une ou plusieurs des revendications précédentes, **caractérisée en ce que** la ceinture de taille (10) dans la configuration pliée dépasse avec une zone de préhension (24) au-delà d'un bord latéral longitudinal (16) de la partie principale de couche (4) dans le sens transversal (14).

15. Couche jetable selon l'une ou plusieurs des revendications précédentes, **caractérisée en ce que** la ceinture de taille (10) présente une étendue dans le sens longitudinal (18) comprise entre 30 et 120 mm, en particulier comprise entre 30 et 100 mm, en particulier comprise entre 30 et 80 mm, en particulier comprise entre 30 et 75 mm, en particulier comprise entre 44 et 70 mm, en particulier comprise entre 40 et 65 mm et en particulier encore comprise entre 40 et 60 mm.

16. Couche jetable selon l'une ou plusieurs des revendications précédentes, **caractérisée en ce que** la ceinture de taille (10) est formée par une section de matière (8) d'un seul tenant.

17. Couche jetable selon l'une des revendications précédentes, **caractérisée en ce que** la ceinture de taille est assemblée de manière inamovible sur une face extérieure (12) de la partie principale (4).

18. Couche jetable selon l'une ou plusieurs des revendications précédentes 16 ou 17, **caractérisée en ce que** la section de matière (8) d'un seul tenant est assemblée sur la partie principale de couche (4) au moyen d'une application de colle (44) en particulier seulement par endroits.
